# NEW EUROPEAN PATENT SPECIFICATION

(11) **EP 3 256 179 B2**
(45) Date of publication and mention of the opposition decision: **18.11.2026**
(45) Mention of the grant of the patent: 20.11.2019
(21) Application number: 16706151.4
(22) Date of filing: 12.02.2016
(51) Int. Cl.: A61L 27/20, A61L 27/52, A61L 27/50

(54) **IMPLANTS FOR SCULPTING, AUGMENTING OR CORRECTING FACIAL FEATURES SUCH AS THE CHIN**
IMPLANTATE ZUR FORMUNG, VERSTÄRKUNG ODER KORREKTUR VON GESICHTSZÜGEN WIE ETWA DEM KINN
IMPLANTS POUR SCULPER, AUGMENTER OU CORRIGER DES TRAITS FACIAUX TELS QUE LE MENTON

(30) Priority: 13.02.2015 WO PCT/FR2015/050357; 16.02.2015 WO PCT/IB2015/000350
(43) Date of publication of application: 20.12.2017
(60) Divisional application: 19205563.0 / 3 653 232
(73) Proprietor: Allergan Industrie, SAS, 74370 Annecy (FR)
(72) Inventor: ROCA MARTINEZ, Jean-Xavier, 74000 Annecy (FR); AYGLON, Aurore, Cruseilles, 74350 (FR)
(74) Representative: Hoffmann Eitle
(86) International application number: PCT/EP2016/053009
(87) International publication number: WO 2016/128550

(56) References cited:
- US-A1- 2006 194 758
- US-A1- 2010 028 437
- Pierre et al.: Dermatol. Surg. 2015, 41, S120-S126;"Basics of Dermal Filler Rheology"
- Derek Jones: "Injectable Fillers: Principles and Practice",Wiley Blackwell, 2010
- Raspaldo, H., J. Cosmet. Laser Ther. 2008; 10:134-142
- MARCOS BORRELL ET AL: "Lift capabilities of hyaluronic acid fillers", JOURNAL OF COSMETIC AND LASER THERAPY, TAYLOR & FRANCIS LTD, ABINGDON, GB, vol. 13, no. 1, 21 January 2011 (2011-01-21), pages 21 - 27, XP008177767, ISSN: 1476-4172, [retrieved on 20110121], DOI: 10.3109/14764172.2011.552609

## Description

The present invention generally relates to injectable compositions and more specifically relates to injectable implants for adding structure and contour to the lower face as defined in the claims.

Dermal fillers are injectable, biocompatible compositions which are well known to correct wrinkles and folds and add volume to the face. Hyaluronic acid (HA) is still considered by many to be one of the most desirable dermal fillers in that it does not pose the risk of an allergic reaction and it is temporary and reversible. The great majority of hyaluronic acid-based dermal fillers have been specifically developed for treating wrinkles and folds in skin. For instance, US 2010/028437 A describes dermal and subdermal fillers based on hyaluronic acids and pharmaceutically acceptable salts thereof which include a therapeutically effective amount of at least on anesthetic agent such as lidocaine. To be useful for facial contouring or substantial volumizing, it would be advantageous to increase the bulking effect of the compositions, also referred to as "lift". It would also be advantageous to maximize resistances of the compositions to shear and normal deformation happening in the soft tissues of the face. One of the drawbacks of maximizing these resistances, for example, elasticity and cohesivity, is that it is expected that in doing so, the viscosity of the compositions will increase to the point that they become difficult to inject with a thin needle.

There is therefore a great need for an injectable HA based implant that is specifically designed to be effective in adding substantial volume to the face, for example, for contouring the lower face, for example, for augmenting or correcting the chin, for example, for correction of chin retrusion, or for example, for augmenting or correcting the nose. It would be highly advantageous if such an implant, despite, its high viscosity, would remain easy to inject with a thin needle.

The shape of the chin has long been recognized as an important feature of the face that elicits a strong aesthetic perception that tends to be associated with personality traits of an individual. A deficient chin that lacks projection is commonly labeled a "weak chin" while prominent chins are labeled "strong chins", both implying strength of personality.

Several studies have suggested that faces with average proportions are viewed as the most attractive and that small features including a small chin are interpreted as attractive in females while the expanded chin and jaw, as a result of maturation, are interpreted as attractive in males. The appearance of the chin is a determinant of perceived attractiveness and can even influence an individual's psychosocial well-being.

Chin augmentation is conventionally performed by surgically placing a permanent implant above the jaw. The procedure is currently among the top aesthetic surgical procedures performed, based on the American Society for Aesthetic Plastic Surgery (ASAPS), and has increased 71% since 2010.

A retrusive chin can be the result of changes in growth of the lower third of the face during maturation, trauma, or facial aging, the latter of which may exacerbate the deformities or asymmetries caused by the former two. The shape of the mandible affects the mouth, chin, and neck. As an individual ages, the reduction in skeletal support of this region makes soft tissue atrophy prominent, exaggerating jowls, decreasing chin protrusion, and making the jawline look weak. Chin deformities are among the most common bony abnormalities of the face, the most common of which is horizontal microgenia characterized by the presence of normal vertical height with a retruded bony chin.

As the mandible and chin make up the framework of the lower face, augmentation methods to treat age-related chin retrusion and contour changes of the chin area or to treat microgenia have been explored for decades. Where the approach in correcting chin retrusion is to add volume, treatment methods have included chin implants, genioplasty, and injection of silicone and semi-permanent fillers, such as polymethylmethacrylate microspheres, and calcium hydroxyapatite. However, all of these treatment methods have drawbacks. For example, chin implants and genioplasty involve painful surgery that may not result in correction of chin retrusion and aesthetic blending of the area. This approach may exacerbate bone resorption and infection, resulting in the need for implant removal. Injection of semi-permanent fillers have trade-offs between volumizing capacity and adverse events associated with semi-permanent fillers.

### SUMMARY OF THE INVENTION

Accordingly, an injectable implant is provided for facial sculpturing, for example, for augmenting, correcting, restoring or creating volume in the chin and other facial features in a human being as defined in the claims.

The present invention provides temporary, reversible, HA-based structural gels as defined in the claims manufactured specifically to provide a safe, minimally invasive method to create facial volume or facial contours. The present implants provide improved volumizing and lift properties relative to other HA-based injectables, due to a combination of mechanical properties including high elasticity and high cohesivity, while still being easily injectable with a thin needle. The present implants may be used for non-therapeutic injection into the subcutaneous and/or supraperiosteal space. In many embodiments the implants are moldable after injection, and therefore permit sculpting, contouring, and shaping across the injected areas, for example, the chin and jaw area.

The implants comprise a composition comprising a hyaluronic acid (HA) crosslinked with 1,4-butanediol diglycidyl ether (BDDE). The compositions are suitable for injection, for example, through a fine gauge needle, and are capable of augmenting, correcting, or creating volume or lift in the face, for example, the lower face, for example, the chin or jaw, or for the midface, for example, the nose.

The HA concentration is greater than 20 mg/g. In some embodiments, the HA concentration is about 21 mg/g, or about 22 mg/g, or about 23 mg/g, or about 24 mg/g, or about 25 mg/g, or about 26 mg/g, or about 27 mg/g, or about 28 mg/g, or about 29 mg/g, or about 30 mg/g or greater. In other embodiments, the composition has an HA concentration of between 22.5 mg/g to 27.5 mg/g, for example, 25.0 mg/g.

In some embodiments the method adds volume and lift to the chin or jawline or nose of the patient for a period of time in the range of about 9 months to about 24 months after the administration or injection into the chin or jawline of the patient. The composition may be moldable, for example, by physical manipulation of the tissue near the implant for a period of time after injection. The compositions may have a setting time, when the composition is no longer moldable and substantially retains its shape for the duration of the implant, within about 24 to about 48 hours after being implanted or injected.

In some embodiments, the compositions further include an anesthetic agent, for example, lidocaine HCl. For example, the compositions may include about 0.3% w/w lidocaine HCl.

In preferred embodiments, the compositions comprise a hyaluronic acid gel, preferably in an amount of about 25 mg; and lidocaine hydrochloride, preferably in an amount of about 3 mg, in a phosphate buffer (pH 7.2), preferably in a volume q.s. 1 mL.

The compositions are made with a mixture of low molecular weight hyaluronic acid and high molecular weight hyaluronic acid. In particular, the crosslinked hyaluronic acid is made from about 50% and about 100% of a low molecular weight hyaluronic acid prior to being crosslinked with the crosslinking agent. In some embodiments, the crosslinked hyaluronic acid is made from about 70% to about 90% of a low molecular weight hyaluronic acid prior to being crosslinked with the crosslinking agent. In some embodiments, the crosslinked hyaluronic acid is made from about 90% of a low molecular weight hyaluronic acid prior to being crosslinked with the crosslinking agent.

Using primarily a low molecular weight HA prior to crosslinking, for example about 50% or greater, for example, about 70% or about 90% low molecular weight HA, rather than using primarily a high molecular weight HA, produces a more robust, longer lasting, moldable hydrogel, having a higher cohesivity and elasticity, and more specifically suitable for facial sculpturing and augmentation by means of subcutaneous or supraperiosteal injection.

In some embodiments, the HA has a degree of crosslinking of between about 4% and about 12%. For example, the HA has a degree of crosslinking of about 4%, or about 6%, or about 8%, or about 10%. In some embodiments the HA has a degree of crosslinking of about 6.5%. In other embodiments, the HA has a degree of crosslinking of about 7.5%, or about 8.5%, or about 9.5%, or about 10.5%.

In another aspect of the invention, non-therapeutic methods for correcting chin retrusion in a patient are provided. The methods generally comprise supraperiostally administering in the chin of the patient, an effective amount of a composition comprising BDDE-crosslinked hyaluronic acid (HA), the HA having a degree of crosslinking of about 10%, and having a HA concentration of greater than 20 mg/g. For example, in a preferred embodiment, the HA concentration is about 25 mg/g.

In a specific embodiment, the compositions comprise low molecular weight hyaluronic acid (NaHA) crosslinked with about 10% BDDE (w/w), and formulated to a concentration of about 25 mg/g with 0.3% lidocaine hydrochloride (w/w) in a phosphate buffer, pH 7.2, and supplied in a 1 mL COC (cyclic olefin copolymer) syringe.

The compositions are extrudable through a fine gauge needle, for example, a needle having a gauge of 25G, 26G, 27G, 28G, 29G or 30G. In a specific embodiment, the needle is a needle of 27 gauge X 13 mm / 27 G1/2 X 26mm.

An extrusion force is the force (in Newtons N) needed to extrude the composition from its syringe at a certain rate. With the supplied 1 mL COC syringe and a TSK 27G X 13mm needle, the extrusion force of the compositions of this invention can be between about 4N and about 15N at 13 mm/min, which is considered as very low. For example, the extrusion force can be between about 7N and about 12N, and preferably between about 8N and about 10 N.

In another aspect of the invention, non-therapeutic methods are provided for contouring or correcting a facial feature, for example, a retruded chin, of an individual. The methods comprise, for example, the step of subdermally administering into a treatment area of the patient, an effective amount, for example, about 1.0 ml, or more, for example, about 2.0 ml or more, for example, about 3.0 ml or more, for example, 4.0 mL, of a composition of the invention. The facial feature to be improved or contoured may be a chin, for example, a retruded chin of a patient. The treatment area may include an area selected from the group consisting of the pogonion, the mentum, the left pre-jowl sulcus, the right pre-jowl sulcus, and the sublabial crease. The treatment may comprise administering the composition into two or more of the treatment areas.

### BRIEF DESCRIPTION OF THE DRAWINGS

Figure 1 shows a facial profile and landmarks for calculating G-Sn-Pog angle of a patient.
Figure 2 shows the Burstone Angle of an average chin.

### DETAILED DESCRIPTION

Certain terms as used in the specification are intended to refer to the following definitions, as detailed below. Where the definition of terms departs from the commonly used meaning of the term, applicant intends to utilize the definitions provided below, unless specifically indicated.

The term "about" in the context of numerical values will be readily understood by a person skilled in the art, and preferably means that specific values may be modified by +/- 10%. As regards endpoints of ranges, the modifier "about" preferably means that the lower endpoint may be reduced by 10% and the upper endpoint increased by 10%. It is also contemplated that each numerical value or range disclosed in this application can be absolute, i.e. that the modifier "about" can be deleted.

All numbers herein expressing "molecular weight" of HA are to be understood as indicating the weight average molecular weight (Mw) in Daltons.

The molecular weight of HA is calculated from an intrinsic viscosity measurement using the following Mark Houwink relation: $Intrinsic Viscosity \left(L/g\right) = 9.78 \times 10 - 5 \times Mw 0.690 .$

The intrinsic viscosity is measured according to the procedure defined by the European Pharmacopoeia (HA monograph N°1472, 01/2009).

Unless stated otherwise, the molecular weight refers to the weight average molecular weight (Mw). The HA used to make the present compositions may comprise a mixture of high molecular weight HA, low molecular weight HA, and/or medium molecular weight HA, wherein the high molecular weight HA has a molecular weight greater than about 2,000,000 Da (or an intrinsic viscosity greater than 2.2 L/g) and wherein the low molecular weight HA has a molecular weight of less than about 1,000,000 Da (or an intrinsic viscosity lower than 1.4 L/g). For example, the high molecular weight HA in the present compositions may have an average molecular weight in the range about 2 MDa to about 4.0 MDa, for example, about 3.0 MDa (2.9 L/g). In another example, the high molecular weight HA may have an average molecular weight of between about 2.4 MDa to about 3.6 MDa, for example, about 3.0 MDa. The high molecular weight HA may have an intrinsic viscosity greater than about 2.2 L/g, for example, between about 2.5 L/g to about 3.3 L/g.

Low molecular weight HA can have a molecular weight of between about 200,000 Da (0.2 MDa) to less than 1.0 MDa, for example, between about 300,000 Da (0.3 MDa) to about 750,000 Da (1.1 L/g), up to but not exceeding 0.99 MDa (1.4 L/g). The low molecular weight HA may have an intrinsic viscosity of less than about 1.40 L/g, for example, between about 0.6 L/g and about 1.2 L/g.

Preferably, there is no overlap between the molecular weight distribution of the low and high molecular weight HA materials.

Preferably, the mixture of the low molecular weight HA and high molecular weight HA has a bimodal molecular weight distribution. The mixture may also have a multi-modal distribution.

In one aspect of the invention, the compositions comprise HA having a high molecular weight component and a low molecular weight component, and the high molecular weight component has a weight average molecular weight at least twice the weight average molecular weight of the low molecular weight component.

"Degree of crosslinking" as used herein refers to the intermolecular junctions joining the individual HA polymer molecules, or monomer chains, into a permanent structure, or as disclosed herein the soft tissue filler composition. Moreover, degree of crosslinking for purposes of the present disclosure is further defined as the percent weight ratio of the crosslinking agent to HA-monomeric units within the crosslinked portion of the HA based composition. It is measured by the weight ratio of crosslinker to HA monomers.

"Uncrosslinked HA" as used herein refers to individual HA polymer molecules that are not crosslinked. Uncrosslinked HA generally remains water soluble. An uncrosslinked HA fraction may optionally also be included in the compositions, for example, to act as a lubricant and facilitate injection into the facial tissues. Such a composition may comprise an uncrosslinked HA fraction where the added uncrosslinked HA is present at a concentration between about 0.1 mg/g and about 3 mg/g. Preferably, the uncrosslinked HA may be present at a concentration between about 0.2 mg/g and about 1.5 mg/g.

In other embodiments, no uncrosslinked HA is present in the gels, or at least no uncrosslinked HA is added to the gels to act as a lubricant.

The compositions described herein display a high level of elasticity, expressed as a value of elastic modulus (G') measured by oscillation rheology with a strain of 0.8%, using a cone-plate system and measured over a range of frequencies. In some embodiments, the elastic modulus of the compositions measured at 5Hz frequency are from about 500 Pa to about 900 Pa. This is considered as high elasticity in the context of HA-based dermal fillers and contributes to the lifting effect by making the implant more resistant to shear deformation.

Cohesivity refers to the capacity of the gel to stay attached to itself, for example, meaning the resistance to cutting and the ability to elongate or compress the gel without it separating into pieces. The cohesivity of the gels according to the present invention can be quantified as follows (cf. Derek Jones "Injectable Filers: Principles and Practice", Wiley, 2011, Chapter 3). A small sample of the gel (e.g. 1 mL) is placed onto the plane surface of a rheometer. The sample is placed such that it forms a little heap. A moveable upper plate is placed onto the sample so that the sample is fully covered, e.g. when looking at the plate in a direction perpendicular to the surface of the rheometer, the sample cannot be seen. In order to ensure this, one must chose a plate size that is larger than the sample size. Ideally, the center of the plate is placed over the sample. Typically, for 1mL of gel material, a 25mm diameter upper plate is used.

In the next step of the measurement, one then adjusts the gap between the moveable plate and the surface to 2.5 mm. While slowly and steadily moving the plate from this initial position towards a gap width of 0.9 mm within 2 min one records the force (Fn) exerted by the sample in normal direction on the plate.

Once a gap width of 0.9 mm is reached, the system is allowed to relax for 12 minutes. During this time, the measurement is continued. Five measurements are done. To normalize the forces measured, all 5 initial Fn values measured when the test starts are averaged (arithmetic mean) and this resulting average is subtracted from all other data points. The maximum force at the end on the compressive part of this test (when reaching the minimal 0.9mm gap width between the upper plate and the plane) is called the compression force and is the characteristic value for determining the cohesivity of the gel.

Specifically, a force of 20 gmf (0.1962 N) or more indicates a cohesive material in the sense of the present invention. Gels with lower compression force values are generally not considered cohesive in the context of the present invention. The accuracy of this measurement is in the order of ±5 gmf. In the context of this invention, the injectable formulation has a high cohesivity of at least about 60 gmf, for example about 60 to about 200 gmf. For example, in a preferred embodiment, cohesivity is between about 60 and about 100 gmf, which will give to the implant a high resistance to pressure and normal forces in the soft tissues of the face.

In the context of a dermal filler, the cohesivity as defined above will contribute to the lift capacity (clinically called the volumizing / bulking effect) provided by the gel clinically, along with its elastic modulus G'. While cohesive gels can show a good volumizing effect, non-cohesive or weakly cohesive materials with a similar elastic modulus exhibits lower lift capacity due to the non-cohesive gel material spreading more than a more cohesive material when submitted to vertical compression. In the context of this invention, the compositions exhibit both high levels of elastic modulus and high levels of cohesivity, to maximize the lifting effect upon implantation.

In certain advantageous, exemplary embodiments, the present implants or fillers generally comprise a cohesive, sterile composition which is implantable subdermally or supraperiostially into the chin area, nose or jawline of the patient in need thereof, for example a patient desiring an improved facial profile or stronger chin. The composition generally comprises a crosslinked hyaluronic acid (HA) crosslinked with 1,4-butanediol diglycidyl ether (BDDE); and the HA concentration of the composition is greater than 20 mg/g. For example, in some embodiments, the HA concentration is about 22.5 mg/g, or about 25 mg/g, or about 27.5 mg/g. The HA used for crosslinking may be made with a mixture of low molecular weight hyaluronic acid and high molecular weight hyaluronic acid. In some embodiments, the compositions have an elastic modulus between about 500 Pa and about 900 Pa at 5Hz, and a cohesivity above about 60 gmf. Advantageously, in some embodiments, the compositions exhibit an extrusion force between about 4N and about 15N, for example, between about 8N and about 10 N, at 13 mm/min using a 1 mL COC syringe and a 27G x 13mm needle.

In one aspect of the invention, injectable HA-based implants having an improved lift capacity, relative to commercial HA-based dermal fillers, are provided. The present implants are, in some instances in the present disclosure, referred alternatively as dermal fillers and subdermal fillers. The implants and fillers of the present invention are based on hyaluronic acids (HA) and pharmaceutically acceptable salts of HA, for example, sodium hyaluronate (NaHA). Methods of making these compositions, and methods of use of these compositions, are also provided.

As used herein, hyaluronic acid (HA) can refer to any of its hyaluronate salts, and includes, but is not limited to, sodium hyaluronate (NaHA), potassium hyaluronate, magnesium hyaluronate, calcium hyaluronate, and combinations thereof. Both HA and pharmaceutically acceptable salts thereof can be used in this invention.

In addition, in embodiments with anesthetics, the concentration of one or more anesthetics is in an amount effective to mitigate pain experienced upon injection of the composition. The at least one local anesthetic can be selected from the group of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethisoquin, dimethocaine, diperodon, dicyclomine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octocaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof. In one embodiment, the at least one anesthetic agent is lidocaine, such as in the form of lidocaine HCl. The compositions described herein may have a lidocaine concentration of between about 0.1% and about 5% by weight of the composition, for example, about 0.2% to about 1.0% by weight of the composition. In one embodiment, the composition has a lidocaine concentration of about 0.3% by weight (w/w %) of the composition. The concentration of lidocaine in the compositions described herein can be therapeutically effective meaning the concentration is adequate to provide a therapeutic benefit without inflicting harm to the patient.

The present compositions may be manufactured by the steps of providing purified HA material for example, in the form of NaHA fibers; the HA material having a desired molecular weight, for example, a mixture of low molecular weight and high molecular weight HA at a desired ratio, hydrating the HA material; and crosslinking the hydrated HA material with a suitable crosslinking agent at the desired ratio to form a crosslinked HA-based gel. The gel may then be neutralized and swollen. If desired, a solution containing lidocaine, preferably an acidic salt of lidocaine chlorohydrate, may be added to form a HA/lidocaine gel. The gel may be homogenized, for example, by beating or mixing with a shear force. The homogenized composition may then be packaged in syringes. The syringes are then sterilized by autoclaving at an effective temperature and pressure. For example, the compositions are sterilized by autoclaving, for example, being exposed to temperatures of at least about 120°C to about 130°C and/or pressures of at least about 82.7 kPa (12 pounds per square inch (PSI)) to about 137.9 kPa (20 PSI) for a period of at least about 1 minute to about 15 minutes. The sterilized syringes are packaged along with a fine gauge needle for use by a physician.

More specifically, the initial raw HA material may comprise fibers or powder of NaHA, for example, bacterial-sourced NaHA fibers. Alternatively, the HA material may be animal derived, for example, from rooster combs. It is contemplated that the HA material may be a combination of raw materials including HA and at least one other polysaccharide, for example, another glycosaminoglycan (GAG).

In one method of manufacturing the compositions, pure, dry NaHA fibers are hydrated in an alkaline solution to produce an uncrosslinked NaHA gel. Any suitable alkaline solution may be used to hydrate the NaHA in this step, for example, but not limited to aqueous solutions containing sodium hydroxide (NaOH), potassium hydroxide (KOH), sodium bicarbonate (NaHCO₃), lithium hydroxide (LiOH), and the like. The resulting alkaline gel will have a pH above 7.5. The pH of the resulting alkaline gel can have a pH greater than 9, or a pH greater than 10, or a pH greater than 12, or a pH greater than 13.

The next step in the manufacturing process may include the step of crosslinking the hydrated, alkaline NaHA gel with a suitable crosslinking agent. The crosslinking agent may be any agent known to be suitable for crosslinking polysaccharides and their derivatives via their hydroxyl groups. One particular suitable crosslinking agent is 1,4-butanediol diglycidyl ether (BDDE).

In another embodiment, the crosslinking of the HA is accomplished during hydration of the HA fibers, by hydrating the combined high and low molecular weight fibers in an alkaline solution containing a crosslinking agent, for example, BDDE.

The degree of crosslinking in the HA component of the present compositions is at least about 4% and is up to about 12% BDDE/HA, w/w, for example, about 10%, for example, about 8%, for example, about 6%, for example, about 4%. In a specific embodiment, the degree of crosslinking is about 6.5%. In some embodiments the HA has a degree of crosslinking of about 6.5%. In other embodiments, the HA has a degree of crosslinking of about 7.5%, or about 8.5%, or about 9.5%, or about 10.5%.

The hydrated crosslinked, HA gels may be swollen to obtain the desired HA concentration. This step can be accomplished by neutralizing the crosslinked, hydrated HA gel, for example by adding an aqueous solution containing of an acid, such as HCl. The gels are then swelled in a phosphate buffered saline (PBS) solution for a sufficient time and at a low temperature.

The gels may now be purified by conventional means such as, dialysis against a phosphate buffer, or alcohol precipitation, to recover the crosslinked material, to stabilize the pH of the material and to remove any un-reacted crosslinking agent. Additional water or a slightly alkaline aqueous solution can be added to bring the concentration of the HA in the composition to a desired concentration. The HA concentration of the compositions is adjusted to above 20 mg/g, for example, to about 25 mg/g. In other embodiments, the HA concentration is adjusted to yield an HA concentration of about 21 mg/g, about 22 mg/g, about 23 mg/g, about 24 mg/g, about 26 mg/g, about 27 mg/g, about 28 mg/g, about 29 mg/g, or about 30 mg/g.

In embodiments in which an anesthetic agent is to be included in the final composition, such as lidocaine, the pH of the purified crosslinked HA gels may be adjusted to cause the gel to become slightly alkaline such that the gels have a pH of accomplished by any suitable means, for example, by adding a suitable amount of dilute NaOH, KOH, NaHCO3 or LiOH, to the gels or any other alkaline molecule, solution and/or buffering composition.

An effective amount of the anesthetic, for example, lidocaine, such as lidocaine HCl, is then added to the purified crosslinked NaHA gels. For example, in some embodiments, the lidocaine HCl is provided in a powder form which is solubilized using water for injection (WFI). The gels are kept neutral with a buffer or by adjustment with diluted NaOH in order that the final HA/lidocaine composition will have a desired, substantially neutral pH. The final compositions including lidocaine may have a lidocaine concentration of between at least about 0.1% and about 5%, for example, about 2% by weight of the composition, or in another example about 0.3%.

After the addition of the lidocaine HCl, or alternatively, during the addition of the lidocaine HCl, the HA/lidocaine gels, or compositions, are homogenized to create highly homogenous HA/lidocaine gels having a desired consistency and stability. Preferably, the homogenization step comprises mixing, stirring, or beating the gels with a controlled shearing force obtaining substantially homogenous mixtures.

After homogenizing the HA composition, an amount of uncrosslinked HA solution or gel may be added to the composition to increase lubricity.

In some embodiments, no solution of uncrosslinked HA is added to the composition after homogenization.

The compositions may then be introduced into syringes and sterilized. Syringes useful according to the present description include any syringe known in the art capable of delivering viscous dermal filler compositions. The syringes generally have an internal volume of about 0.4 mL to about 3 mL, more preferably between about 0.5 mL and about 1.5 mL or between about 0.8 mL and about 2.5 mL. This internal volume is associated with an internal diameter of the syringe which plays a key role in the extrusion force needed to inject high viscosity dermal filler compositions. The internal diameters are generally about 4 mm to about 9 mm, more preferably from about 4.5 mm to about 6.5 mm or from about 4.5 mm to about 8.8 mm. Further, the extrusion force needed to deliver the HA compositions from the syringe is dependent on the needle gauge. The gauges of needles used generally include gauges between about 18G and about 40G, more preferably about 25G to about 33G, or from about 25G to about 30G. For example, in some embodiments, the compositions are packaged in a 1 mL syringe and injected using a 27 G needle.

One preferable method of sterilization of the filled syringes is by autoclave. Autoclaving can be accomplished by applying a mixture of heat, pressure and moisture to a sample in need of sterilization. Many different sterilization temperatures, pressures and cycle times can be used for this step. For example, the filled syringes may be sterilized at a temperature of at least about 120°C to about 130°C or greater. Moisture may or may not be utilized. The pressure applied is in some embodiments depending on the temperature used in the sterilization process. The sterilization cycle may be at least about 1 minute to about 20 minutes or more.

Another method of sterilization incorporates the use of a gaseous species which is known to kill or eliminate transmissible agents. Preferably, ethylene oxide is used as the sterilization gas and is known in the art to be useful in sterilizing medical devices and products.

A further method of sterilization incorporates the use of an irradiation source which is known in the art to kill or eliminate transmissible agents. A beam of irradiation is targeted at the syringe containing the HA composition, and the wavelength of energy kills or eliminates the unwanted transmissible agents. Preferable energy useful include, but is not limited to ultraviolet (UV) light, gamma irradiation, visible light, microwaves, or any other wavelength or band of wavelengths which kills or eliminates the unwanted transmissible agents, preferably without substantially altering of degrading the HA composition.

Preferably, the present compositions also remain stable when stored for long periods of time. For example, many of the present compositions have a shelf life of about 6 months, about 12 months, about 18 months, or about 24 months or greater, when stored at a temperature between about 2 to 25 degrees C. In a specific embodiment, the compositions are stable at a temperature of between 2 to 25 degrees C for a period of at least 18 months. In another specific embodiment, the compositions are stable at a temperature or between 2 to 25 degrees C for a period of at least 24 months.

The technique for injection of the present compositions may vary with regard to the angle and orientation of the bevel, and the quantity administered. In general, the present compositions are injected subcutaneously and/or supraperiosteally to increase chin projection, limiting treatment to the pogonion, the mentum (inferior aspect of the chin), pre-jowl sulci (left and right), and sublabial (mental) crease to achieve optimal correction and aesthetic chin contour. The appropriate injection volume will be determined by the investigator but is generally not to exceed a maximum total volume of about 4.0 mL for initial and top-up treatments combined. Up to about 4.0 mL total is allowed for repeat treatment. No more than about 2.0 mL is permitted to be injected into a single treatment area at any treatment session, where treatment areas are defined as the pogonion, the mentum, the pre-jowl sulci (left and right), and the sublabial (mental) crease.

Prior to injection of the present compositions, the treatment area has to be thoroughly disinfected to ensure that there is no contamination of the injectable filler with bacteria or a foreign body (e.g., make-up, talc from gloves).

Next, the 27G 1/2"/27 G x 13 mm needle supplied should be attached to the syringe (according to Directions for Use). Prior to injecting the present compositions, the plunger rod has to be depressed until the product visibly flows out of the needle and wipe any excess on sterile gauze.

The present compositions are injected as follows: Inject the present compositions slowly, and observe the skin for signs of colour change or discolouration. Observe the subject for pain or discomfort. Inject the present compositions in a smooth and measured manner. Insert the needle being mindful of the local vascular anatomy at the injection site. Aspirate to ensure there is no blood backflow to suggest an intravascular location of the tip of the needle.

Pogonion may be injected supraperiosteally using multiple small boluses. Mentum may be injected supraperiosteally using multiple small boluses. Pre-jowl sulci (left and right) may be injected using a deep subcutaneous fanning technique. Sublabial (mental) crease may be injected using linear, retrograde or anterograde superficial subcutaneous threading.

When treatment is completed, the treated site may be gently massaged to assure that the product is evenly distributed and conforms to the contour of the surrounding tissues. If overcorrection occurs, gently massage the area between your fingers or against an underlying bone to obtain optimal results.

The present compositions are not to be injected into the blood vessels (intravascular). Introduction of hyaluronic acid into the vasculature may occlude the vessels and could cause infarction or embolization. Symptoms of vascular occlusions and embolization include pain that is disproportionate to the procedure or remote to the injection site, immediate blanching that extends beyond the injected area and that may represent vascular tributary distribution, and colour changes that reflect ischemic tissue such as a dusky or reticular appearance.

Injecting the product too superficially or in large volumes over a small area may result in visible and persistent lumps and/or discoloration.

When using a retrograde technique, inject the present compositions applying even pressure on the plunger rod while slowly pulling the needle backward. It is important that the injection be stopped just before the needle passes the subcutaneous/dermal interface to prevent material from leaking out or ending up too superficially in the skin. When using an anterograde technique, be sure the needle is in the subcutaneous tissue before the injection is started.

If the needle is blocked, do not increase the pressure on the plunger rod but stop the injection and replace the needle.

If the treated area is swollen immediately after the injection, an ice pack may be applied to the site for a short period. If subjects report inflammatory reactions which persist for more than 1 week, or any other side effect which develops, the medical practitioner should use an appropriate treatment.

In preferred embodiments, the present compositions comprise a hyaluronic acid gel, preferably in an amount of about 25 mg; and lidocaine hydrochloride, preferably in an amount of about 3 mg, in a phosphate buffer (pH 7.2), preferably in a volume q.s. 1 mL, prefilled in e.g. a 1 mL single-use syringe, wherein the hyaluronic acid gel is crosslinked with BDDE. This prefilled e.g. 1 mL single-use syringe may be contained in a kit (blister pack) along with two single use needles (e.g. 27G 1/2"/27 G x 13 mm needles) . The content of the syringe may be sterilised by moist heat. The single-use needles may be sterilised by radiation.

The present compositions are injectable implants intended for restoration and creation of facial volume, e.g. in the chin and jaw area. The presence of lidocaine is meant to reduce the subject's pain during treatment.

### EXAMPLE 1

### MANUFACTURE OF AN INJECTABLE IMPLANT IN ACCORDANCE WITH AN EMBODIMENT OF THE INVENTION

Predried fibers of sodium hyaluronate (NaHA) (0.9 g) having a molecular weight of about 0.9 MDa is weighed out into a first receptacle.

Predried fibers of NaHA (0.1 g) having a molecular weight of about 3.0 MDa is weighed out into a second receptacle.

The two different grades of NaHA are combined and diluted into a 1% sodium hydroxide solution and mixed for one to two hours at between 20°C and 50°C to obtain a substantially homogenous, alkaline HA gel.

In a separate receptacle, the chosen crosslinking agent, 1,4-butanediol diglycidyl ether (BDDE), is diluted into a 1% sodium hydroxide solution to a final concentration of 10% BDDE (wt/wt).

To the alkaline HA gel was added 10% (wt/wt) BDDE (1 g of the previously prepared BDDE solution). The resulting mixture is mechanically homogenized.

The mixture is then maintained at 50°C for 3 to 4.5 hours.

The resulting crosslinked HA polymer is then immersed in a phosphate buffer (PB) containing hydrochloric acid to stabilize the pH.

The crosslinked HA polymer so obtained is then immersed in baths of phosphate buffer to remove unreacted crosslinking agent and HA, providing the purified hydrogel, wherein the degree of crosslinking is about 6.5%.

Optionally, dry HA material having a high molecular weight is hydrated in 1 liter of phosphate buffer to obtain an uncrosslinked HA gel. This uncrosslinked HA gel can be added to the crosslinked HA composition to represent up to 5% (w/w) of the total HA concentration.

The hydrogel obtained is then homogenized mechanically to ensure the final homogeneity, and packed into syringes which are sterilized in an autoclave.

The gel obtained is an injectable composition that can be administered subdermally or supraperiostally through a fine gauge needle (e.g. 27 Gauge). The composition is useful for restoring, contouring, or creating facial volume, for example, in the chin, jaw area, or nose of a person, as described elsewhere herein.

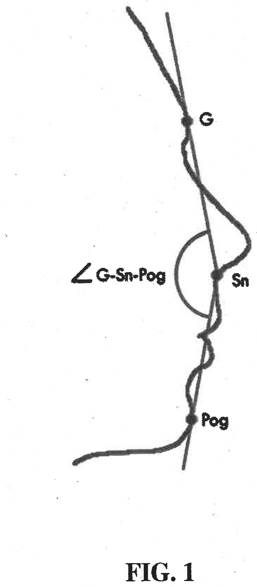

In one aspect of the invention, non-therapeutic methods are provided for improving a patient's facial profile. For example, in some embodiments, methods are provided for changing a person's G-Sn-Pog facial angle, for example, for increasing a person's G-Sn-Pog facial angle. For example, in some embodiments, methods of treatment are provided for correcting chin retrusion in a patient. In some embodiments of the invention, the patient treated an initial pre-treatment G-Sn-Pog facial angle of less than about 165°. After the treatment, the patient has an increased G-Sn-Pog facial angle, that is, a facial angle greater than the initial pre-treatment facial angle. In one embodiment, the patient has a G-Sn-Pog angle of about 169° or greater after the step of administering. The G-Sn-Pog angle may be measured using conventional equipment and calculations, for example, may be based on calculations of facial angle derived from digital images of the patient, for example, using Canfield scientific facial imaging equipment. Figure 1 shows facial profile and landmarks for calculating G-Sn-Pog angle of a patient, which can be used to diagnose or determine the presence and/or degree of chin retrusion, using know methods.

The methods generally comprise administering into at least one treatment area of the face of the patient, an effective amount of a composition comprising BDDE-crosslinked hyaluronic acid (HA), the HA having a degree of crosslinking of about 6.5%, or about10%, and having a HA concentration of greater than 20 mg/g.

In some embodiments, non-therapeutic treatment methods are provided, the methods comprising supraperiostally administering a composition, such as described herein, into at least one treatment area of the face of a patient, wherein the patient has a G-Sn-Pog facial angle of 145° to 165°. The facial angle value may be based on calculations of facial angle derived from digital images of the patient, or using other techniques. In accordance with some embodiments, the step of administering results in the patient having an increased G-Sn-Pog angle relative to the patient's G-Sn-Pog facial angle prior to the treatment, for example, immediately prior to the administering step. In some embodiments, the patient has an increased G-Sn-Pog angle for a period of time in the range of at least about 3 months, or more preferably, for at least about 6 months, for example, for about 9 months to about 24 months, after the step of administering. For example, the patient has an increased G-Sn-Pog angle for at least about 6 months, or for at least about 9 months, or for at least about 12 months or for at least about 18 months or for at least about 24 months for after the step of administering.

In some embodiments, the treatment area is an area selected from the group consisting of the pogonion, the mentum, the left pre-jowl sulcus, the right pre-jowl sulcus, and the sublabial crease. The treatment may comprise administering the composition into two or more of the treatment areas. The administration comprises supraperiostally or subdermally injecting the compositions in an amount of between about 0.5 mL and about 3.0 mL per treatment area. In some embodiments, the amount injected into a given treatment area is no greater than 2.0 mL. In some embodiments, the total amount injected in a single treatment session, over all treatment areas, is between 2.0 mL to about 6.0 mL, for example, about 2.5 mL, about 3.0 mL, about 3.5 mL. about 4.0 mL, about 4.5 mL, about 5.0 mL, about 5.5 mL, or about 6.0 mL. In some embodiments, the amount administered into a single treatment session is about 4.0 mL or less.

### Restoration and Creation of Volume in the Chin and Jaw

In one aspect, the present invention provides non-therapeutic methods for restoring and creating volume in the chin and jaw, for example, in sculpting, shaping, and contouring across specific treatment areas of the face. The treatment areas may include one or more of the pogonion (the most projecting point on the anterior surface of the chin), mentum, (the lowest point on the chin), left and right pre-jowl sulci (left antigonion notch and right antigonion notch), and sublabial (mental) crease (the crease between the lower lip and the mentum).

The shape and projection of the chin contribute to the proportional balance of the face that underlies attractiveness. A chin lacking projection is commonly labeled a "weak chin" whereas prominent chins are labeled "strong chins" and imply strength of personality. Several studies have suggested that faces with average proportions are viewed as the most attractive and that juvenile features including a small chin are interpreted as attractive in females while a strong chin and jaw are interpreted as attractive in males. The appearance of the chin is a determinant of perceived attractiveness and can influence an individual's psychosocial well being

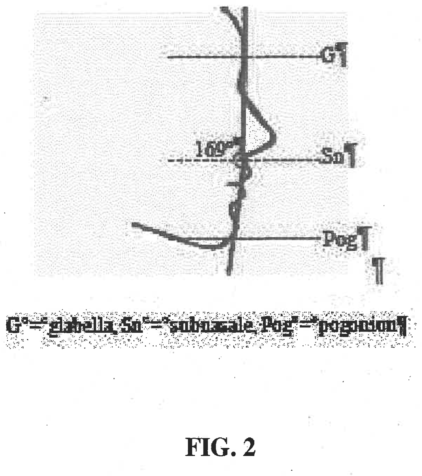

Average proportions are dictated by analysis of a representation of facial profiles in a population and include the distances and angles between the nose, lip, and mentum. Several soft tissue landmarks have been used in cephalometric analysis to measure and diagnose chin protrusion and retrusion deviations from average facial parameters. The intersection of the upper facial and anterior lower facial components and the angle formed by the point on the glabella, subnasale, and pogonion (G-Sn-Pog) has been extensively analyzed to understand the average chin projection common among populations. The Burstone angle (Figure 2) has been defined as approximately 169° for the average chin, and the approximate angle (168° to 169°) has been confirmed in several studies.

Incrementally, deviations from the average chin result in the perception of facial unattractiveness. Analyzing the relationship between facial profile and perception of attractiveness shows that chin prominence plays a major role in this perception. To understand the relationship between the degree of chin prominence and attractiveness, a series of profile images altered in 2-mm increments from an idealized profile image was presented to a group of pretreatment orthognathic patients, clinicians, and laypeople. Subjects were asked to rate each image on a 7-point Likert scale ranging from extremely unattractive to extremely attractive. Ratings of perceived attractiveness decreased an average of 0.15 on the Likert Scale for each 2 mm of chin retrusion and were apparent after 4 mm of change. The degree of chin retrusion at which surgery was desired was 11 mm for patients and clinicians and 10 mm for laypeople. The most attractive image was that which displayed an ideal orthognathic profile with the soft tissue pogonion resting on the true vertical line.

### EXAMPLE 2

### METHOD FOR INCREASING THE G SN POG FACIAL ANGLE IN A SUBJECT HAVING CHIN RETRUSION OR A WEAK CHIN

A composition of the invention is administered as an injectable implant, by subdermal or supraperiosteal injection in the chin and/or jaw area of a 32 year old male subject. The subject complains he has a "weak chin". The doctor measures the subject's facial angle and determines that the a G-Sn-Pog angle of about 150°, which is substantially lower than the classic Burstone angle of the average chin (approximately 169°). The measurement is based on calculations of facial angle derived from digital images obtained using Canfield imaging equipment and software.

The doctor considers the subject's chin/jaw retrusion to be amenable to correction with a treatment goal consistent with increasing chin projection horizontally (in the profile view), not chin lengthening or widening.

The doctor believes that he can provide the subject with a more attractive facial profile and a stronger jawline by using the implantable compositions described herein.

The subject undergoes three treatment sessions, including initial treatment, top-up treatment, and repeat treatment, as described below.

For each treatment, the treatment areas include at least one or more of the following treatment areas: the pogonion (the most projecting point on the anterior surface of the chin), the mentum (the lowest point on the chin), the left pre-jowl sulcus (left antigonion notch), the right pre-jowl sulcus (right antigonion notch), and/or the sublabial crease (the crease between the lower lip and the mentum).

The doctor implants no more than 2.0 mL into a single treatment area at any of the treatment sessions.

The initial treatment is performed on the subject as follows. The doctor uses aseptic skin preparation and administers anesthesia following his standard practice. The application of ice and topical anesthesia may reduce injection discomfort. Injectable anesthesia is limited to the treatment areas only is and administered with certainty not to distort the planned treatment areas.

Using needles (27 gauge x 13 mm / 27G ½") supplied with a kit, the doctor injects the compositions described herein subcutaneously and/or supraperiosteally to increase chin projection (horizontally in the profile view), as well as to aesthetically sculpt, contour, and shape, limiting treatment to the pogonion, mentum, pre-jowl sulci, and sublabial (mental) crease. Suitable injection techniques have been described above. The treatment goal is to increase chin projection (horizontally in the profile view) and achieve aesthetic chin contour. The doctor determines the appropriate injection volume up to about 4.0 mL for initial and possible top-up treatments combined.

The doctor gently molds the treated area using manual manipulation of the overlying tissue to achieve the desired facial contour.

A top-up treatment occurs approximately 30 days after the initial treatment if desired by the subject, or if in the doctor's opinion, optimal (full) increase in chin projection and/or aesthetic contouring was not achieved by the initial treatment. If a top-up treatment is performed, the volume of the administered composition as a combined total (initial treatment and top-up treatment) is between about 2.0 mL to about 4.0 mL.). During this visit, the doctor evaluates the treatment areas for any localized reaction and discusses any reported symptoms. 3D facial digital images (frontal and profile images) are captured for objective calculation of the angle of chin retrusion. If the doctor determines at top-up follow-up visit that optimal (full) increase in chin projection or aesthetic contouring was not achieved after the initial treatment, then subject is advised that he may receive a top-up treatment.

A single repeat treatment is administered at a scheduled visit between months 18 and 24 if repeat treatment is warranted in the doctor's opinion and/or is desired by the subject. Injection volume for the chin does not exceed a total volume of 4.0 mL for the repeat treatment.

Although the invention has been described and illustrated with a certain degree of particularity, it is understood that the present disclosure has been made only by way of example.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention.

## Claims

1. A sterile composition, implantable subdermally or supraperiostially into the chin area, jawline or nose of a patient in need thereof, the composition comprising a crosslinked hyaluronic acid (HA) crosslinked with 1,4-butanediol diglycidyl ether (BDDE);
wherein the HA concentration of the composition is greater than 20 mg/g;
wherein the HA used for crosslinking is made with a mixture of low molecular weight hyaluronic acid and high molecular weight hyaluronic acid,
where the HA used for crosslinking is a mixture containing at least 50% by weight of low molecular weight HA, based on the total weight of the HA;
wherein the composition has an elastic modulus between 500 Pa and 900 Pa at 5Hz;
wherein the composition has a cohesivity above 60 gmf;
wherein the composition exhibits an extrusion force between 4N and 15N at 13 mm/min using a 1 mL COC syringe and a 27G x 13mm needle.

2. The composition of claim 1 where the HA concentration is 22.5 mg/g, 25 mg/g, or 27.5 mg/g.

3. The composition of any one of claims 1-2 where the HA used for crosslinking is a mixture containing at least 70% by weight of low molecular weight HA, preferably at least 90% of low molecular weight HA, based on the total weight of the HA.

4. The composition of any one of claims 1-3 wherein (i) the cohesivity is between 60 gmf and 200 gmf or between 60 gmf and 100 gmf; (ii) the extrusion force is between 7 and 12N at 13 mm/min using a 1 mL COC syringe and a 27G x 13mm needle or between 8N and 10N at 13 mm/min using a 1 mL COC syringe and a 27G x 13mm needle.

5. The composition of any one of claims 1-4 wherein the composition further includes an anesthetic agent such as lidocaine hydrochloride, wherein the composition preferably includes 0.3% by weight lidocaine hydrochloride, based on the total weight of the composition.

6. The composition of any one of claims 1-5 wherein the HA has a degree of crosslinking of between 4% and 10%, such as 4%, 6%, 8%, 10% or 6.5%.

7. The composition of any one of claims 1-6 wherein the composition comprises 25 mg of crosslinked HA and 3 mg of lidocaine in a phosphate buffer pH 7.2 q.s. 1 mL, and wherein the crosslinked HA preferably has a degree of crosslinking of 6.5%.

8. A kit, comprising the composition according to any one of claims 1-7, prefilled in a single-use syringe such as a 1 mL COC syringe, and at least one single-use needle such as a 27G x 13mm needle.

9. A non-therapeutic method for facial sculpturing, such as for augmenting, correcting, restoring or creating volume in the chin and jaw area, in a patient comprising:
subcutaneously and/or supraperiosteally administering into at least one treatment area of the face of the patient, an effective amount of the composition of any one of claim 1-7 by injection of said composition; and
optionally massaging the treatment area of the patient's face, after administering is completed, to assure that the product conforms to the contour of the surrounding tissues;
wherein, prior to administering said composition, the treatment area is optionally disinfected;
the treatment area being preferably selected from the group consisting of the pogonion, the mentum, the left pre-jowl sulcus, the right pre-jowl sulcus, and the sublabial (mental) crease.

## Patentansprüche

1. Sterile Zusammensetzung, die subdermal oder supraperiostal in den Kinnbereich, die Kieferlinie oder die Nase eines Patienten, der diese benötigt, implantiert werden kann, wobei die Zusammensetzung eine mit 1,4-Butandioldiglycidylether (BDDE) vernetzte Hyaluronsäure (HA) umfasst;
wobei die HA-Konzentration der Zusammensetzung größer als 20 mg/g ist;
wobei die zur Vernetzung verwendete HA aus einer Mischung aus Hyaluronsäure mit niedrigem Molekulargewicht und Hyaluronsäure mit hohem Molekulargewicht hergestellt wird, wobei die zur Vernetzung verwendete HA eine Mischung ist, die mindestens 50 Gew.-% HA mit niedrigem Molekulargewicht, auf der Grundlage des Gesamtgewichts der HA, enthält;
wobei die Zusammensetzung einen Elastizitätsmodul zwischen 500 Pa und 900 Pa bei 5 Hz aufweist;
wobei die Zusammensetzung eine Kohäsivität von mehr als 60 gmf aufweist;
wobei die Zusammensetzung eine Extrusionskraft zwischen 4 N und 15 N bei 13 mm/min unter Verwendung einer 1-ml-COC-Spritze und einer 27 G x 13 mm-Nadel aufweist.

2. Zusammensetzung nach Anspruch 1, wobei die HA-Konzentration 22,5 mg/g, 25 mg/g oder 27,5 mg/g beträgt.

3. Zusammensetzung nach einem der Ansprüche 1-2, wobei die zur Vernetzung verwendete HA eine Mischung ist, die mindestens 70 Gew.-% HA mit niedrigem Molekulargewicht, bevorzugt mindestens 90 Gew.-% HA mit niedrigem Molekulargewicht, auf der Grundlage des Gesamtgewichts der HA, enthält.

4. Zusammensetzung nach einem der Ansprüche 1-3, wobei (i) das Kohäsionsvermögen zwischen 60 gmf und 200 gmf oder zwischen 60 gmf und 100 gmf beträgt; (ii) die Extrusionskraft zwischen 7 und 12 N bei 13 mm/min unter Verwendung einer 1-ml-COC-Spritze und einer 27 G x 13 mm-Nadel oder zwischen 8 und 10 N bei 13 mm/min unter Verwendung einer 1-ml-COC-Spritze und einer 27 G x 13 mm-Nadel liegt.

5. Zusammensetzung nach einem der Ansprüche 1-4, wobei die Zusammensetzung weiter ein Anästhetikum wie Lidocainhydrochlorid einschließt, wobei die Zusammensetzung bevorzugt 0,3 Gew.-% Lidocainhydrochlorid, auf der Grundlage des Gesamtgewichts der Zusammensetzung, einschließt.

6. Zusammensetzung nach einem der Ansprüche 1-5, wobei die HA einen Vernetzungsgrad zwischen 4 % und 10 %, wie 4 %, 6 %, 8 %, 10 % oder 6,5 %, aufweist.

7. Zusammensetzung nach einem der Ansprüche 1-6, wobei die Zusammensetzung 25 mg vernetzte HA und 3 mg Lidocain in einem Phosphatpuffer mit einem pH-Wert von 7,2, q.s. 1 ml, umfasst, wobei die vernetzte HA bevorzugt einen Vernetzungsgrad von 6,5 % aufweist.

8. Kit, umfassend die Zusammensetzung nach einem der Ansprüche 1-7, vorgefüllt in einer Einwegspritze wie einer 1-ml-COC-Spritze, und mindestens eine Einwegnadel wie beispielsweise eine 27 G x 13 mm-Nadel.

9. Nicht-therapeutisches Verfahren zur Gesichtsformung, wie z. B. zur Verstärkung, Korrektur, Wiederherstellung oder Schaffung von Volumen im Kinn- und Kieferbereich, bei einem Patienten, umfassend:
subkutanes und/oder supraperiosteales Verabreichen einer effektiven Menge der Zusammensetzung nach einem der Ansprüche 1-7 durch Injektion der Zusammensetzung in mindestens einen Behandlungsbereich des Gesichts des Patienten; und
wahlweise, Massieren des Behandlungsbereichs des Gesichts des Patienten nach Abschluss der Verabreichung, um sicherzustellen, dass sich das Produkt an die Kontur des umgebenden Gewebes anpasst;
wobei vor der Verabreichung der Zusammensetzung der Behandlungsbereich wahlweise desinfiziert wird;
wobei der Behandlungsbereich bevorzugt aus der Gruppe ausgewählt wird, die aus dem Pogonion, dem Mentum, der linken Pre-Jowl-Furche, der rechten Pre-Jowl-Furche und der sublabialen (mentalen) Falte besteht.

## Revendications

1. Composition stérile, implantable sous le derme ou avant le périoste dans la zone du menton, de la mâchoire ou du nez d'un patient qui en a besoin, la composition comprenant un acide hyaluronique réticulé (HA) réticulé avec un 1,4-butanediol diglycidyléther (BDDE) ;
dans laquelle la concentration en HA de la composition est supérieure à 20 mg/g ;
dans laquelle l'HA utilisé pour la réticulation est fabriqué avec un mélange d'acide hyaluronique de faible poids moléculaire et d'acide hyaluronique de poids moléculaire élevé, où l'HA utilisé pour la réticulation est un mélange contenant au moins 50 % en poids d'HA de faible poids moléculaire, sur la base du poids total de l'HA ;
dans laquelle la composition présente un module élastique compris entre 500 Pa et 900 Pa à 5 Hz ;
dans laquelle la composition présente une cohésivité supérieure à 60 gmf ;
dans laquelle la composition présente une force d'extrusion comprise entre 4 N et 15 N à 13 mm/min en utilisant une seringue en COC de 1 mL et une aiguille de 27 G x 13 mm.

2. Composition selon la revendication 1, où la concentration en HA est de 22,5 mg/g, 25 mg/g ou 27,5 mg/g.

3. Composition selon l'une quelconque des revendications 1 et 2 où l'HA utilisé pour la réticulation est un mélange contenant au moins 70 % en poids d'HA de faible poids moléculaire, préférablement au moins 90 % d'HA de faible poids moléculaire, par rapport au poids total de l'HA.

4. Composition selon l'une quelconque des revendications 1 à 3, dans laquelle (i) la cohésivité est comprise entre 60 gmf et 200 gmf ou entre 60 gmf et 100 gmf ; (ii) la force d'extrusion est comprise entre 7 et 12 N à 13 mm/min en utilisant une seringue en COC de 1 mL et une aiguille de 27 G x 13 mm ou entre 8 N et 10 N à 13 mm/min en utilisant une seringue en COC de 1 mL et une aiguille de 27 G x 13 mm.

5. Composition selon l'une quelconque des revendications 1 à 4, dans laquelle la composition inclut en outre un agent anesthésique tel que le chlorhydrate de lidocaïne, dans laquelle la composition inclut préférablement 0,3 % en poids de chlorhydrate de lidocaïne, par rapport au poids total de la composition.

6. Composition selon l'une quelconque des revendications 1 à 5, dans laquelle l'HA présente un degré de réticulation compris entre 4 % et 10 %, tel que 4 %, 6 %, 8 %, 10 % ou 6,5 %.

7. Composition selon l'une quelconque des revendications 1 à 6, dans laquelle la composition comprend 25 mg d'HA réticulé et 3 mg de lidocaïne dans un tampon phosphate de pH 7,2 q.s. 1 ml, et dans laquelle l'HA réticulé présente préférablement un degré de réticulation de 6,5 %.

8. Kit comprenant la composition selon l'une quelconque des revendications 1 à 7, prérempli dans une seringue à usage unique telle qu'une seringue en COC de 1 ml, et au moins une aiguille à usage unique telle qu'une aiguille de 27 G x 13 mm.

9. Procédé non thérapeutique de sculpture faciale, tel que pour augmenter, corriger, restaurer ou créer du volume dans la zone du menton et de la mâchoire, chez un patient comprenant :
l'administration sous-cutanée et/ou avant le périoste dans au moins une zone de traitement du visage du patient, d'une quantité efficace de la composition selon l'une quelconque des revendications 1 à 7 par injection de ladite composition ; et
le massage facultatif de la zone de traitement du visage du patient, après que l'administration est terminée, pour s'assurer que le produit se conforme au contour des tissus environnants ;
dans lequel, avant l'administration de ladite composition, la zone de traitement est facultativement désinfectée ;
la zone de traitement étant préférablement sélectionnée dans le groupe consistant en le pogonion, le mentum, le sillon avant-bajoue gauche, le sillon avant-bajoue droit et le pli sous-labial (relatif au menton).
